# EUROPEAN PATENT APPLICATION

(11) **EP 2 829 610 A1**
(43) Date of publication of application: **28.01.2015**
(21) Application number: 13763936.5
(22) Date of filing: 22.03.2013
(51) Int. Cl.: C12P 7/62, C02F 3/30

(54) **METHOD FOR OBTAINING POLYHYDROXYALKANOATES FROM BEER-INDUSTRY WASTEWATER**

(30) Priority: 23.03.2012 ES 201230438
(71) Applicant: Universidade Da Coruña, 15071 A Coruña (ES)
(72) Inventor: VEIGA BARBAZAN, Mª Del Carmen, 15071 A Coruña (ES); KENNES, Christian, 15071 A Coruña (ES); BEN GARCIA, Marta, 15071 A Coruña (ES)
(74) Representative: Carvajal y Urquijo, Isabel
(86) International application number: PCT/ES2013/070189
(87) International publication number: WO 2013/140013

(57) **Abstract**

An obtainment method for polyhydroxyalkanoates from beer-industry waste water. An obtainment method for polyhydroxyalkanoate that consists of three stages: acidogenic fermentation of the beer-industry waste effluent in a reactor to obtain volatile fatty acids, feeding the fermented effluent obtained in the previous stage into a reactor that contains a mixed culture of microorganism with the object of selecting a biomass with a high polyhydroxyalkanoate accumulation capacity, and discontinuously, via the addition of consecutive pulses, feeding the fermented effluent obtained in the first stage to a reactor that contains biomass selected in second stage.

## Description

### FIELD OF INVENTION

The present invention is a microbial production method for a bioplastic from beer-industry waste water. Said method is related to bioengineering and the plastics industry.

### BACKGROUND OF THE INVENTION

The intensive and wide use of plastics in our society is faced with two fundamental problems: on the one hand, manufacturing depends on oil, a non-renewable natural resource, and on the other hand, the environmental impact caused due to the resistance to biodegradation.

Currently, materials with similar properties but that degrade easily are being investigated with the aim of solving the problems that come from using conventional plastics. Among these are bioplastics and biopolymers. The term bioplastic or biopolymer essentially encompasses three types of polymer: chemically synthesised polymers, biodegradable plastics based on starch (which are obtained by mixing starch and a plastic, for example, polyethylene) and polyhydroxyalkanoates.

Polyhydroxyalkanoates (PHA) are 100% biodegradable polymers, since they are synthesised as reserve material by a large number of Gram-positive and Gram-negative bacteria belonging to more than 75 different genus. These polyhydroxyalkanoates provide a good alternative to plastics derived from oil since they have properties similar to polyethylene and polypropylene.

Polyhydroxyalkanoates are considered to be of great industrial interest as biodegradable plastics and they are used in different areas: packaging materials (for example, containers, films, etc.), pharmaceutical industry, medicine, food industry, cosmetic industry and agriculture.

PHAs are lineal polymers of 3-hydroxy acids in which the carboxyl group of a monomer forms an ester type bond with the hydroxyl group of the following monomer.

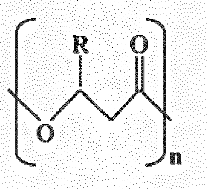

The composition of the lateral chain or R atom determines the identity of the monomer unit.

Currently, approximately 125 hydroxyalcanoic acids are known, which form part of PHAs (identified in diverse bacterial genus). Among these, 3-hydroxyalkanoics with 3 to 14 carbon atoms may be found with a wide variety of linear or branched chain saturated groups including aliphatic or aromatic lateral groups.

The present invention uses the biomass (a mixed culture) as an inoculum and beer-industry waste water as a substrate.

The beer industry generates effluents in the grape-juice manufacturing, fermentation, beer maturing, filtration, pre-packaging and packaging processes, which gives rise to a large quantity of waster liquid that must be treated before being poured into the environment.

These waste waters have a high content of organic material that degrades easily, mainly fatty acids, ethanol and sugars.

Currently, the commercial production of PHA is based in methods that use pure micro-organism cultures, which entails an enormous expense in sterilisation and expensive substrates. PHA production for pure cultures takes place when an essential nutrient, with the exception of the carbon source, acts as a limiter for growth.

Some methods for the production of polyhydroxyalkanoates are known in the state of the art. EP1400569 proposes a system for the production of biopolymers from the use of waste water as a substrate and mixed cultures as an inoculum. The system consists of 2 stages. The first stage is an acidogenic stage during which the complex organic material in the medium is degraded to obtain simpler molecules such as volatile fatty acids. The resulting effluent from this first stage is fed through stage 2. Stage 2 is an aerobic stage developed in a sequencing reactor (*Sequencing Batch Reactor,* SBR). During this stage, a mixed culture is fed with the effluent that comes from the first stage under dynamic feeding conditions.

However, a three-stage process applied to a specific type of waste water, beer-industry effluent, with different operating conditions for each of the stages in the process is proposed in the present application. EP1400569 proposes the operation of the reactor at high organic fill rates (OFR), above 20 g COD/L.d (g COD/litre.day, COD being the chemical oxygen demand), applying short operation cycles of 2 hours. However, the present application proposes working with beer water at an OFR of 3.6 g COD/L.d, using 12 hour operation cycles. An OFR of 3.6 g COD/L.d is equivalent to 100 Cmmol/ L.d (mmol of carbon/litre.day).

WO2011/073744 suggests the production of polyhydroxyalkanoates in three stages from the effluent of a paper industry and an aerobic sludge enriched with filamentous bacteria that has a high accumulation capacity. Essential nutrient limiting concentrations are applied (C/N/P 100/4.4/1.3 relation), of an essential micronutrient and low concentrations of oxygen (a concentration no higher than 2 mg/L) with the object of enriching the filamentous microorganism sludge. However, the present application suggests a very different C/N/P relation, of between 11/1.7/1 and 50/5/1, and is not limited to the oxygen concentration supplied, the airflow rate being 1 vvm (volume of air per volume of liquid per minute).

The problem that the art suggests is providing an optimised method for obtaining polyhydroxyalkanoates with mixed cultures, which has yields that enable said methods to be competitive in relation to the obtainment methods of polyhydroxyalkanoates with pure cultures.

### DESCRIPTION OF THE INVENTION

The present invention is an obtainment method for polyhydroxyalkanoate, which comprises the following stages:
a) acidogenic fermentation of the beer-industry waste effluent in a reactor to obtain volatile fatty acids, characterised in that the hydraulic retention time is between 1 and 1.6 days, the temperature is between 30 and 37 °C and the pH is 6.0,
b) feeding the fermented effluent obtained in stage a) into a reactor that contains a mixed microorganism culture with the object of selecting a biomass with a high polyhydroxyalkanoate accumulation capacity, characterised in that it is operated in alternate periods of substrate absence and excess of 12 hours, the carbon/nitrogen/phosphorous relation is between 11/1.7/1 and 50/5/1, the organic fill rate is between 100 and 120 Cmmol/L·d, the solids retention time is between 6 and 10 days, the airflow is 1 vvm and the temperature is 30°C,
c) discontinuously feeding, via the addition of consecutive pulses, the fermented effluent obtained in stage a) to a reactor that contains biomass selected in stage b), characterised in that the airflow is 1 vvm and the temperature is 30°C.

The first stage of the method of the invention consists of acidogenic fermentation during which the complex organic material in the beer-industry waste water is transformed, preferably in SBR reactors, into simpler molecules such as volatile fatty acids. The volatile fatty acids are the substrate (fermented effluent) that is used for the subsequent production of polyhydroxyalkanoates.

The fermented waste effluent in the acidgenic reactor is subsequently used as a substrate in both stage b) for enriching the mixed culture of polyhydroxyalkanoate accumulating bacteria and stage c) for producing the biopolymer.

The enrichment of the mixed culture PHA accumulating bacteria takes place in the second stage of the method of the invention. In order to favour the accumulation of PHA, condition that limit the growth of bacteria are applied. The limiting conditions consist of subjecting the microorganisms to alternate periods of substrate absence and excess. Subjected to these conditions, the bacteria accumulate the PHA as intercellular reserves, which they may use as a substrate or as an energy source. In this enriching stage, the effluent produced in the previous acidogenic fermentation stage is used.

Both the enriching stage of the mixed culture and the polyhydroxyalkanoate production stages are preferably carried out in SBR reactors. SBRs enable alternate substrate excess/absence conditions to be applied (aerobic dynamic feeding conditions or ADF) that favour the physiological adaptation of the sludge, thus favouring the polymer accumulation.

It is possible to favour the development of biomass with high polyhydroxyalkanoate accumulation capacity via the control of operation conditions and the operational parameters. During the sludge enriching stage, macronutrients (nitrogen and phosphorous) are added in a concentration that is sufficient so that the latter are not limiting, in this way the growth of biomass from the polymer accumulated during the substrate absence stage is favoured. The bacteria with high accumulation capacity have a significant competitive advantage over those with low accumulation capacity, thus achieving the enrichment of the cultures with a high capacity to accumulate polyhydroxyalkanoates. In the present invention, the C/N/P relation is between 11/1.7/1 y 50/5/1.

SRT also plays an important role in the selection of a sludge. However, in the event that complex substrates are used, it must not fix at values that are too low, given that the complexity of the substrate may slow the kinetics of the method. In such a case, low SRT lead to low biomass concentrations at the start of the cycle, which favours the increase of time in which the substrate is present in the medium. The longer the substrate remains in the medium, the more it favours the development of bacteria with low accumulation capacity; these suffer a physiological adaptation in which growth is favoured over the accumulation of internal reserves. In the present invention, SRT has values comprised between 6 and 10 days.

The airflow is also a key parameter, since insufficient airflow may favour the development of a biomass with poor decanting. It is important to not limit the oxygen in the growth medium. In the present invention, the airflow is 1 vvm (volume of air per volume of liquid per minute).

The temperature in stage b) of the present invention is 30 °C, since said temperature favours the kinetics of the process, thus enabling the substrate to be consumed quicker and the regime of substrate presence/absence is re-established. Another measure adopted that favours re-establishment of the substrate presence/absence regime is the use of long cycle times (12 hours).

Lastly, in the third stage of the method of the invention these sludges enriched with PHA accumulation bacteria are discontinuously fed an effluent with a high volatile fatty acid content to transform them into bioplastic. The substrate is added in various pulses to avoid possible substrate inhibition.

A preferred embodiment is the method of the invention where in stage a) the hydraulic retention time is 1.6 days and the temperature is 30 °C.

Another preferred embodiment is the method of the invention where in stage b) the carbon/nitrogen/phosphorous relation is 11/1.7/1, the organic filling rate is 100 Cmmol/L·d and the solids retention time is 6 days.

An embodiment is the polyhydroxyalkanoate obtained according to the method of the invention, where said polyhydroxyalkanoate contains 70% hydroxybutyrate and 30% hydroxyvalerate.

### PREFERRED EMBODIMENTS

### Example 1. Production of polyhydroxyalkanoates is a reactor of the SBR type fed with beer-industry waste water.

Firstly, the waste water of the beer industry is treated in an acidogenic reactor of the SBR type. The acidogenic fermentation is carried out at a Hydraulic Retention Time (HRT) of 1.6 days, temperature of 30 °C and a control pH of 6.0. The acidification percentage reached was 60%, having the following composition in fatty acids: 60% acetic, 16% propionic, 12% butyric and 12% n-valeric. The total COD of fed waste water oscillates between 4000-6000 mg/L.

The enrichment of the mixed culture of the aerobic stage of the process is carried out in a SBR reactor with a usable volume of 1 L operating under aerobic dynamic feeding conditions. The SBR is operated with a 12 hour cycle, being distributed in the following stages: aeration stage (10.8 hours), decanting stage (1 hour) and emptying stage (0.2 hours). During the first two minutes of the aeration stage, the medium unloaded from the reactor was replaced with previously fermented beer water. The reactor is operated with a HRT and SRT of 1 and 6 days, respectively. The temperature of the reactor is controlled at 30 °C by means of a thermostatic bath and the airflow is regulated at 1 vvm via the use of a rotameter. The pH is not controlled, although it is monitored. The OFR applied to the reactor was 100 Cmmol/L·d. Along with the feeding and by means of another peristaltic pump, a concentrated solution of ammonium chloride and potassium dihydrogen phosphate with the object of maintaining the C/N/P relation at 11/1.7/1. Likewise, thiourea was added to the medium in order to inhibit nitrification and so that all the nitrogen added to the medium is used for cellular growth.

During the continuous operation of the reactor accumulation yields to the order of 0.5 Cmmol/Cmmol are achieved under these conditions. Furthermore, the substrate consumption and polymer production rates were relatively high, 1.35 Cmmol/CmmolX·h (PHA carbon mmol/active biomass mmol·hour) and 0.35 Cmmol/CmmolX·h (PHA carbon mmol/active biomass mmol·hour), respectively.

The reactor was operated for a period of 3 times the SRT, time for which it is considered that the reactor reached a steady state. The stationary state is characterised by obtaining reproducible feast times (time in which the substrate is present in the medium), a constant biomass concentration and a constant variation in the parameters of the system.

Once the reactor was in a steady state, several batch tests were carried out with the object of testing the capacity of the sludge selected under these conditions. While these tests were carried out, several substrate pulses containing a volatile fatty acid concentration of 50 Cmmol/L were added, whilst nutrients were not added. The remaining operating conditions were kept the same as in the continuous operation of the reactor.

The enriched biomass under these conditions has a PHA contents by dry cell weight of 50-60%. The polymer obtained with this substrate under the conditions described was a copolymer containing hydroxybutyrate and hydroxyvalerate units at a ratio of 70%:30%.

## Claims

1. An obtainment method for polyhydroxyalkanoates, which comprises the following stages:
a) acidogenic fermentation of the beer-industry waste effluent in a reactor to obtain volatile fatty acids, **characterised in that** the hydraulic retention time is between 1 and 1.6 days, the temperature is between 30 and 37 °C and the pH is 6.0,
b) feeding the fermented effluent obtained in stage a) into a reactor that contains a mixed microorganism culture with the object of selecting a biomass with a high polyhydroxyalkanoate accumulation capacity, **characterised in that** it is operated in alternate periods of substrate absence and excess of 12 hours, the carbon/nitrogen/phosphorous relation is between 11/1.7/1 and 50/5/1, the organic fill rate is between 100 and 120 Cmmol/L·d, the solids retention time is between 6 and 10 days, the airflow is 1 vvm and the temperature is 30°C,
c) discontinuously feeding, via the addition of consecutive pulses, the fermented effluent obtained in stage a) to a reactor that contains biomass selected in stage b), **characterised in that** the airflow is 1 vvm and the temperature is 30°C.

2. The method according to claim 1, where in stage a) the hydraulic retention time is 1.6 days and the temperature is 30°C.

3. The method according to claims 1 or 2, where in stage b) the carbon/nitrogen/phosphorous relation is 11/1.7/1, the organic fill rate is 100 Cmmol/L·d and the solids retention time is 6 days.

4. A polyhydroxyalkanoate obtained according to any of the claims 1 to 3, **characterised in that** said polyhydroxyalkanoate contains 70% hydroxybutyrate and 30% hydroxyvalerate.
